# EUROPEAN PATENT SPECIFICATION

(11) **EP 3 095 439 B1**
(45) Date of publication and mention of the grant of the patent: **17.07.2019**
(21) Application number: 14878522.3
(22) Date of filing: 04.08.2014
(51) Int. Cl.: A61K 8/81, A61K 8/06, A61K 8/41, A61Q 19/00, A61Q 19/08, A61Q 5/00, A61Q 19/02

(54) **EMULSION COMPOSITION FOR SKIN**
EMULSIONSZUSAMMENSETZUNG FÜR HAUT
COMPOSITION D'ÉMULSION POUR LA PEAU

(30) Priority: 17.01.2014 JP 2014007190
(43) Date of publication of application: 23.11.2016
(73) Proprietor: Otsuka Pharmaceutical Co., Ltd., Tokyo 101-8535 (JP)
(72) Inventor: KAKO, Junko, Osaka-shi Osaka 540-0021 (JP); OGIHARA, Miyoko, Osaka-shi Osaka 540-0021 (JP); SUMA, Momoko, Osaka-shi Osaka 540-0021 (JP)
(74) Representative: dompatent von Kreisler Selting Werner - Partnerschaft von Patent- und Rechtsanwälten mbB
(86) International application number: PCT/JP2014/070524
(87) International publication number: WO 2015/107709

(56) References cited:
- EP-A1- 2 308 459
- EP-A1- 2 444 058
- WO-A1-2011/077779
- JP-A- H10 182 348
- JP-A- 2001 058 914
- JP-A- 2005 068 023
- JP-A- 2007 009 019
- JP-A- 2007 169 240
- JP-A- 2009 286 757

## Description

### Technical Field

The present invention relates to a skin emulsion composition.

### Background Art

Many polymer-containing preparations are known. Polymers have particular features, such as thickening, dispersion stability, emulsion stability, film formation, and solubilization, depending on their type. Polymers are thus considered to be an important component playing a role in imparting desired properties to preparations (Non-patent Literature 1). However, a high polymer content in a preparation is likely to make the preparation, for example, sticky, spoiling the feeling of use.

Some examples of polymers used in preparations include polyacrylic acids and alkyl-modified carboxyvinyl polymers. Although polyacrylic acids and alkyl-modified carboxyvinyl polymers are used to thicken preparations, the thickening effect of these polymers is not entirely satisfactory. Because of the insufficient thickening effect, these polymers may be added in an increased amount to enhance the effect. However, as mentioned above, a high polymer content in a preparation is likely to compromise the feeling of use of the preparation. It is therefore not an effective way to simply add a higher amount of these polymers to enhance the thickening effect.

### Citation List

### Non-patent Literature

Non-patent Literature 1: Keshohin Jiten, The Society of Cosmetic Chemists of Japan, published by Maruzen, September 25, 2004, second issue, pp.177 to 181

### Summary of Invention

### Technical Problem

The present inventors uniquely focused on the idea that if the thickening effect produced by polyacrylic acids or alkyl-modified carboxy polymers is further increased, viscosity can be efficiently imparted to a preparation without adding an excessive amount of these polymers, providing the preparation with a further improved feeling of use, which is likely to be spoiled by, for example, stickiness, while retaining the desired viscosity.

An object of the present invention is to provide a skin emulsion composition with a further improved thickening effect produced by a polyacrylic acid and an alkyl-modified carboxy polymer. Another object of the invention is to provide a means to further improve the thickening effect produced by a polyacrylic acid or an alkyl-modified carboxy polymer.

### Solution to Problem

The present inventors conducted extensive research to achieve the objects, and found that applying a compound represented by formula (I) described below to a polyacrylic acid salt and an alkyl-modified carboxy polymer can increase the thickening effect produced by the polyacrylic acid salt and the alkyl-modified carboxy polymer in a skin emulsion composition. The inventors conducted further research based on these findings and completed the invention described below.

### (I) Emulsion Composition for Skin

Item 1. A skin emulsion composition comprising the following components (1) to (4), and having a viscosity of 10,000 mPa·s or more, with a BM-type viscometer using a roter No. 4 at 12 rpm for 60 seconds,
   (1) at least one member selected from the group consisting of polyacrylic acids and salts thereof,
   (2) an alkyl-modified carboxy polymer,
   (3) a compound represented by the following formula (I) 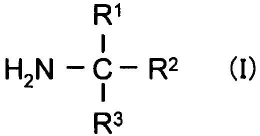 wherein R¹, R², and R³ are the same or different and represent C₁₋₃ alkyl or C₁₋₃ alkyl substituted with at least one hydroxyl group, with the proviso that at least one of R¹, R², and R³ is C₁₋₃ alkyl substituted with at least one hydroxyl group; and
   (4) an oil in an amount of 10% by weight or more of the composition.
Item 2. The skin emulsion composition according to item 1, wherein the oil is at least one member selected from the group consisting of waxes, solid hydrocarbon oils, solid ester oils, liquid hydrocarbon oils, liquid ester oils, plant oils, animal oils, and silicone oils.
Item 3. The skin emulsion composition according to Item 1 or 2, comprising ethanol in an amount of 20% by weight or less.
Item 4. The skin emulsion composition according to any one of Items 1 to 3, wherein component (1) and component (2) are each present in an amount of 2% by weight or less of the composition.
Item 5. The skin emulsion composition according to any one of Items 1 to 4, which has a pH of 7 or less.
Item 6. The skin emulsion composition according to any one of Items 1 to 5, wherein component (1) is at least one member selected from the group consisting of polyacrylic acid sodium salts, polyacrylic acid potassium salts, polyacrylic acid ammonium salts, polyacrylic acid triethylamine salts, polyacrylic acid triethanolamine salts, polyacrylic acid arginine salts, polyacrylic acid aminomethyl propanol salts, and polyacrylic acid aminomethyl propanediol salts.
Item 7. The skin emulsion composition according to any one of Items 1 to 6, wherein component (2) is an acrylic acid/alkyl methacrylate copolymer.
Item 8. The skin emulsion composition according to any one of Items 1 to 7, wherein component (3) is at least one member selected from the group consisting of aminomethyl propanol, aminomethyl propanediol, and amino hydroxymethyl propanediol.
Item 9. The skin emulsion composition according to any one of Items 1 to 8, comprising substantially no surfactant other than an acrylic acid-based surfactant.
Item 10. The skin emulsion composition according to any one of Items 1 to 9 excluding compositions for application to scalp hair and/or scalp.
Item 11. The skin emulsion composition according to any one of Items 1 to 10, further comprising an electrolyte, the composition being for use in moisturizing skin or preventing wrinkles on skin.

### (II) Viscosity Increasing Method

Item 1. A method for increasing the viscosity of a skin emulsion composition comprising the following components (1), (2) and (4), the method comprising adding the following component (3) to the skin emulsion composition,
   (1) at least one member selected from the group consisting of polyacrylic acids and salts thereof,
   (2) an alkyl-modified carboxy polymer,
   (3) a compound represented by the following formula (I) 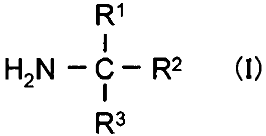 wherein R¹, R², and R³ are the same or different and represent C₁₋₃ alkyl or C₁₋₃ alkyl substituted with at least one hydroxyl group, with the proviso that at least one of R¹, R², and R³ is C₁₋₃ alkyl substituted with at least one hydroxyl group; and 4) an oil in an amount of 10% by weight or more of the composition.
Item 2. The method according to Item 1, wherein the thickened skin emulsion composition has a viscosity of 10,000 mPa·s or more.
Item 3. The method according to Item 1 or 2, wherein the thickened skin emulsion composition comprises ethanol in an amount of 20% by weight or less.
Item 4. The method according to any one of Items 1 to 3, wherein component (1) and component (2) are each present in an amount of 2% by weight or less of the thickened skin emulsion composition.
Item 5. The method according to any one of Items 1 to 4, wherein the thickened skin emulsion composition has a pH of 7 or less.
Item 6. The method according to any one of Items 1 to 5, wherein component (1) is at least one member selected from the group consisting of polyacrylic acid sodium salts, polyacrylic acid potassium salts, polyacrylic acid ammonium salts, polyacrylic acid triethylamine salts, polyacrylic acid triethanolamine salts, polyacrylic acid arginine salts, polyacrylic acid aminomethyl propanol salts, and polyacrylic acid aminomethyl propanediol salts.
Item 7. The method according to any one of Items 1 to 6, wherein component (2) is an acrylic acid/alkyl methacrylate copolymer.
Item 8. The method according to any one of Items 1 to 7, wherein component (3) is at least one member selected from the group consisting of aminomethyl propanol, aminomethyl propanediol, and amino hydroxymethyl propanediol.
Item 9. The method according to any one of Items 1 to 8, wherein the oil is at least one member selected from the group consisting of waxes, solid hydrocarbon oils, solid ester oils, liquid hydrocarbon oils, liquid ester oils, plant oils, animal oils, and silicone oils.

### Advantageous Effects of Invention

The present invention can increase the thickening effect produced by at least one member selected from the group consisting of polyacrylic acids and salts thereof and an alkyl-modified carboxy polymer in a skin emulsion composition by applying a compound represented by formula (I) to the at least one member selected from the group consisting of polyacrylic acids and salts thereof and the alkyl-modified carboxy polymer. Even when the skin emulsion composition contains an electrolyte, which is likely to decrease the viscosity, the present invention can also increase the thickening effect produced by at least one member selected from the group consisting of polyacrylic acids and salts thereof and an alkyl-modified carboxy polymer in the skin emulsion composition by applying the compound represented by formula (I).

### Brief Description of Drawings

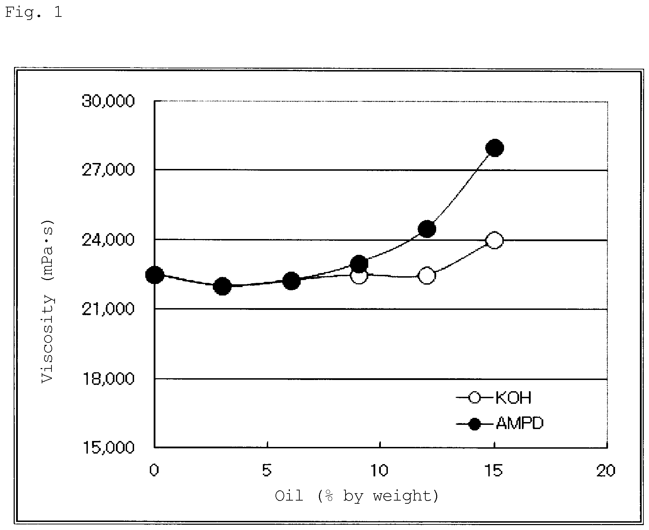

Fig. 1: Fig. 1 shows that the use of aminomethyl propanediol in compositions emulsified with an oil clearly increased the viscosity.

### Description of Embodiments

The following describes the present invention in more detail.

### (I) Emulsion Composition for Skin

The present invention provides a skin emulsion composition comprising (1) at least one member selected from the group consisting of polyacrylic acids and salts thereof, (2) an alkyl-modified carboxy polymer, (3) a compound represented by the following formula (I) 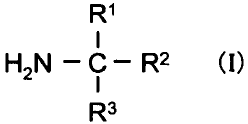 wherein R¹, R², and R³ are the same or different and represent C₁₋₃ alkyl or C₁₋₃ alkyl substituted with at least one hydroxyl group, with the proviso that at least one of R¹, R², and R³ is C₁₋₃ alkyl substituted with at least one hydroxyl group, and (4) an oil in an amount of 10% by weight or more of the composition with the viscosity of the composition being 10,000 mPa·s or more.

The at least one member selected from the group consisting of polyacrylic acids and salts thereof (1) includes polyacrylic acids, polyacrylic acid sodium salts, polyacrylic acid potassium salts, polyacrylic acid ammonium salts, polyacrylic acid triethylamine salts, polyacrylic acid triethanolamine salts, polyacrylic acid arginine salts, polyacrylic acid aminomethyl propanol salts, and polyacrylic acid aminomethyl propanediol salts, and these polyacrylic acid salts are those in which carboxylic acids are in the form of salts listed above. For example, component (1) is preferably a polyacrylic acid salt, and more preferably a cross-linked polyacrylic acid salt.

For component (1), commercially available products may be used, and examples of commercially available products include the Lubrajel Series (ISP (Japan) Ltd), Viscomate Series (Showa Denko K.K.), and Rheogic and Junlon Series (Toagosei Co., Ltd.).

These may be used singly or in a combination of two or more.

The amount of component (1) is not limited as long as the effect of the present invention is obtained. For example, component (1) is present in an amount of 0.001 to 2% by weight, preferably 0.1 to 1% by weight, more preferably 0.1 to 0.5% by weight, and still more preferably 0.2 to 0.4% by weight of the skin emulsion composition.

The alkyl-modified carboxy polymer (2) includes an alkyl-modified carboxyvinyl polymer, more preferably an acrylic acid/alkyl methacrylate copolymer, and still more preferably an acrylic acid/alkyl methacrylate copolymer with a molecular weight of 100,000 to 5,000,000. Preferable examples of such acrylic acid/alkyl methacrylate copolymers include acrylic acid/alkyl methacrylate copolymers with the carbon number of alkyl being 8 to 35, and more preferable examples include acrylic acid/alkyl methacrylate copolymers with the carbon number of alkyl being 10 to 30 (e.g., acrylates/C₁₀₋₃₀ alkyl acrylate crosspolymer).

For component (2), commercially available products may be used. Examples of commercially available products include Pemulen TR-1, Pemulen TR-2, Carbopol Ultrez21, Carbopol Ultrez20, Carbopol 1382, and Carbopol ETD2020 (e.g., The Lubrizol Corporation).

These may be used singly or in a combination of two or more.

The amount of component (2) is not limited as long as the effect of the present invention is obtained. For example, component (2) is present in an amount of 0.01 to 2% by weight, preferably 0.1 to 1% by weight, and more preferably 0.4 to 1% by weight of the skin emulsion composition.

The amount of component (1) relative to the amount of component (2) is not limited as long as the effect of the present invention is obtained. For example, component (1) is present in an amount of preferably 0.1 to 5 parts by weight, more preferably 0.2 to 2 parts by weight, and still more preferably 0.3 to 1 part by weight, per part by weight of component (2).

The compound (3) represented by formula (I) is described by the following structure formula 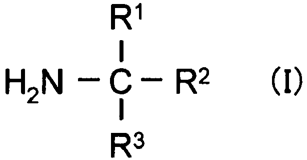 wherein R¹, R², and R³ are the same or different and represent C₁₋₃ alkyl or C₁₋₃ alkyl substituted with at least one hydroxyl group, with the proviso that at least one of R¹, R², and R³ is C₁₋₃ alkyl substituted with at least one hydroxyl group.

Preferable examples of compound (3) represented by formula (I) include those wherein R¹, R², and R³ are the same or different and represent C₁₋₃ alkyl or C₁₋₃ alkyl substituted with 1 to 3 hydroxyl groups, with the proviso that at least one of R¹, R², and R³ is C₁₋₃ alkyl substituted with 1 to 3 hydroxyl groups.

More preferable examples of compound (3) represented by formula (I) include aminomethyl propanol, aminomethyl propanediol, and amino hydroxymethyl propanediol. These can be described, respectively, as 2-amino-2-methyl-1-propanol, 2-amino-2-methyl-1,3-propanediol (AMPD), and 2-amino-2-hydroxymethyl-1,3-propanediol.

These may be used singly or in a combination of two or more.

The amount of component (3) is not limited as long as the effect of the present invention is obtained, preferably as long as the pH of the obtained skin emulsion composition falls within the range of mild acidic to neutral, and more preferably as long as the pH of the obtained skin emulsion composition is 7 or less. For example, component (3) is present in an amount of preferably 0.1 to 10% by weight, and more preferably 0.2 to 2% by weight of the skin emulsion composition.

The amount of component (3) relative to the amount of component (2) is also not limited as long as the effect of the present invention is obtained. For example, component (3) is present in an amount of preferably 0.5 to 10 parts by weight, and more preferably 0.5 to 5 parts by weight, per part by weight of component (2). For example, when component (2) is present in an amount of 0.3 to 2 parts by weight per part by weight of component (1), component (3) is present in an amount of 0.5 to 3 parts by weight, and preferably 1 to 2 parts by weight, per part by weight of component (2).

The viscosity of the skin emulsion composition of the present invention is 10,000 mPa·s or more, preferably 10,000 to 50,000 mPa·s, more preferably 15,000 to 45,000 mPa·s, and still more preferably 20,000 to 40,000 mPa·s at 25°C immediately after the composition has been prepared.

In the present invention, the viscosity of the skin emulsion composition is measured by a B-type viscometer. The viscosity of 10,000 to 45,000 mPa·s is measured at 25°C with a BM rotor No. 4 (Tokyo Keiki Inc.,) at 12 rpm for 60 seconds, and the viscosity of 45,000 mPa·s or more is measured at 25°C with a Brookfield RV rotor NO.6 at 10 rpm for 60 seconds.

It is sufficient that the pH of the skin emulsion composition of the present invention be typically within the range of mild acidic to neutral. For example, the composition to be applied to the skin (including fingernails, the same applies hereinafter) preferably has a pH of 4 to 7, and more preferably a pH of 5.5 to 7.

The skin emulsion composition of the present invention may optionally comprise, in addition to the components described above, any other pharmaceutically or cosmetically acceptable components. Examples of other components include solvents, electrolytes, preservatives, antioxidants, anti-inflammatory agents, algefacients, fragrances, oil-soluble UV absorbers, chelating agents, neutralizers, pigments, coloring materials, lubricants, emulsion stabilizers, antifoaming agents, protectants, film-forming agents, pH adjusters, thickening agents, sugar, amino acids, vitamins, and surfactants.

The oils of compound (4) include waxes, such as candelilla wax, carnauba wax, hydrogenated jojoba oil, rice bran wax, shellac, lanolin, beeswax, whale wax, and montan wax; solid hydrocarbon oils, such as ozokerite, ceresin, paraffin, polyethylene wax, microcrystalline wax, and vaseline; solid ester oils, such as myristyl myristate, cetyl palmitate, cetyl lactate, cholesteryl oleate, phytosteryl oleate, cholesteryl stearate, and cholesteryl hydroxystearate; liquid hydrocarbon oils, such as squalane, liquid paraffin, liquid isoparaffin, hydrogenated polyisobutene, and α-olefin oligomer; liquid ester oils, such as isopropyl myristate, octyldodecyl myristate, oleyl oleate, decyl oleate, hexyldecyl isostearate, hexyldecyl dimethyl octanoate, isopropyl palmitate, hexyl laurate, butyl stearate, and isostearyl neopentanoate; plant oils, such as avocado oil, almond oil, olive oil, sesame oil, camellia sasanqua oil, safflower oil, soybean oil, castor oil, camellia oil, corn oil, rapeseed oil, rice bran oil, persic oil, palm kernel oil, palm oil, sunflower oil, grape seed oil, cottonseed oil, macadamia nut oil, and theobroma grandiflorum seed oil; animal oils, such as turtle oil, mink oil, and yolk fatty oil; and silicone oils, such as cyclopentasiloxane, dimethicone, aminopropyl dimethicone, (dimethicone/vinylmethicone) crosspolymers, methylphenyl polysiloxane, methyl hydrogen polysiloxane, and octamethyl cyclotetrasiloxane. These may be used singly or in a combination of two or more.

The oil is present in an amount of 10% by weight or more, preferably 10 to 40% by weight, more preferably 10 to 30% by weight, and particularly more preferably 15 to 25% by weight of the skin emulsion composition.

The solvent includes water, such as purified water, distilled water, ion-exchanged water, and natural water; and ethanol, glycerol, propylene glycol, dipropylene glycol, butylene glycol, pentylene glycol, and diglycerol. These may be used singly or in a combination of two or more.

The amount of the solvent is also not limited as long as the effect of the present invention is obtained. A person skilled in the art can suitably determine the amount. Although not limited as long as the effect of the present invention is obtained, the amount of ethanol is preferably a predetermined amount or less from the standpoint of the feeling of use in the skin, particularly skin irritancy. Although the amount of ethanol is not limited as long as the effect of the present invention is obtained, ethanol is present, for example, in an amount of preferably 20% by weight or less, more preferably 0 to 15% by weight, and still more preferably 0.1 to 10% by weight of the skin emulsion composition.

The electrolyte includes inorganic salts, purine-based compounds, water-soluble vitamins, derivatives thereof, and salts thereof. Examples of inorganic salts include chlorides and sulfides of metals, such as sodium, potassium, magnesium, and calcium. The "purine-based compounds" collectively refer to a variety of derivatives having a purine nucleus as the skeleton, and salts thereof. The purine-based compounds include adenine, guanine, deaminated compounds thereof, adenosine, guanosine, inosine, phosphate esters of adenosine (e.g., adenosine monophosphates (e.g., adenosine 2'-monophosphate, adenosine 3'-monophosphate, and adenosine 5'-monophosphate), adenosine diphosphates (e.g., adenosine 5'-diphosphate), adenosine triphosphates (e.g., adenosine 5'-triphosphate), adenosine-3',5'-cyclic phosphate), phosphate esters of guanosine (e.g., guanosine monophosphate, guanosine diphosphate, and guanosine triphosphate), and phosphate esters of inosine (e.g., inosine monophosphate, inosine diphosphate, and inosine triphosphate). The salts thereof are not particularly limited as long as they are pharmaceutically or cosmetically acceptable. Examples include alkali metal salts, such as sodium salts and potassium salts, alkaline-earth metal salts, basic amino acid salts, ammonium salts, and alkanolamine salts. Examples of water-soluble vitamins and derivatives thereof include ascorbic acid, B vitamins, lipoic acid, and derivatives thereof. Specific examples of derivatives of ascorbic acid include magnesium ascorbyl phosphate, sodium ascorbyl phosphate, disodium ascorbyl sulfate, glucoside ascorbate, glucosamine ascorbate, and dehydroascorbic acid. The salts are not particularly limited as long as they are pharmaceutically or cosmetically acceptable, and examples include alkali metal salts, such as sodium salts and potassium salts, alkaline-earth metal salts, basic amino acid salts, ammonium salts, and alkanolamine salts. These electrolytes may be used singly or in a combination of two or more.

The amount of the electrolyte is also not limited as long as the effect of the present invention is obtained. For example, the electrolyte is present in an amount of 0.1 to 10% by weight, and preferably 0.5 to 5% by weight of the skin emulsion composition.

Examples of preservatives or antioxidants include tocopherol, dibutyl hydroxy toluene, soybean extract, pentylene glycol, catechins, benzoic acid, isopropyl methyl phenol, salicylic acid, para-hydroxybenzoic acid esters, and phenoxyethanol.

Examples of anti-inflammatory agents include glycyrrhizic acid, salts thereof, glycyrrhetinic acid, fatty acid esters thereof, tranexamic acid, bromelain, and chamomilla recutita extracts. Examples of chelating agents include ethylene diamine, ethylenediaminetetraacetic acid, ethylenediamine-N,N'-disuccinic acid, and etidronic acid.

Examples of algefacients or fragrances include Mentha (menthol) and essential oils.

Examples of oil-soluble UV absorbers include ethylhexyl methoxycinnamate, octocrylene, diethylamino hydroxybenzoyl hexyl benzoate, polysilicone-15, homosalate, t-butyl methoxydibenzoylmethane, ethylhexyl salicylate, ethylhexyl triazone, bis-ethylhexyloxyphenol methoxyphenyl triazine, methylene bisbenzotriazolyl tetramethylbutylphenol, oxybenzone-3, and drometrizole trisiloxane.

To further increase viscosity, plant-derived thickening agents, such as agar, guar gum, xanthan gum, glucomannan, and alginic acid may optionally be added. Adding these components is effective in further increasing the viscosity of the skin emulsion composition of the present invention. In particular, when the preparation of the present invention is used with a pump container, the thickening agent is present in an amount of preferably 1% by weight or less, more preferably 0.01 to 0.5% by weight, and still more preferably 0.05 to 0.2% by weight of the composition from the standpoint of the feeling of use, such as stringiness. However, a high content of a plant-derived thickening agent is not preferable from the antiseptic viewpoint. Thus, the amount of the thickening agent preferably falls within the ranges described above. When a preparation containing a thickening agent is packed in a pump container, the viscosity is, for example, 20,000 mPa·s or more, preferably 20,000 to 45,000 mPa·s, and still more preferably 25,000 to 40,000 mPa·s at 25°C.

Examples of surfactants include non-ionic surfactants, anionic surfactants, cationic surfactants, and ampholytic surfactants. However, to relieve skin irritancy, it is preferable in the present invention that the composition does not substantially contain, as any other optional components, surfactant(s) other than polyacrylic acids or salts thereof, or that the composition contains surfactant(s) other than polyacrylic acids or salts thereof in an amount of 0.2% by weight or less.

These components may also be used singly or in a combination of two or more.

The skin emulsion composition of the present invention is prepared as an emulsion composition that can be externally applied to the skin. For example, the skin emulsion composition of the present invention can be prepared as an externally applied preparation in a desired form, such as a paste, a mousse, an emulsion, a suspension, a cream, an ointment, a sheet, an aerosol, a spray, and a liniment, by adding components (1) to (4), optionally with other components. Compositions in such forms can be prepared in accordance with a typical procedure in the art.

The skin emulsion composition of the present invention can be used primarily in cosmetic compositions such as basic skin care compositions including milky lotions, creams, lotions, and packs, externally applied pharmaceuticals, or externally applied quasi-drugs.

The skin emulsion composition of the present invention is applied to human skin. The skin emulsion composition of the present invention is not particularly limited in the amount and frequency of application. It is sufficient, for example, to apply a suitable amount of the composition to the skin of the whole body or an intended part of the body once or several times a day, depending on the type or concentration of the contained components, user age, sex, degree of symptoms, form of application, and level of expectation. The skin emulsion composition of the present invention is preferably applied to a part other than scalp hair and/or scalp.

The present invention can increase the thickening effect produced by at least one member selected from the group consisting of polyacrylic acids and salts thereof and an alkyl-modified carboxy polymer in a skin emulsion composition by applying a compound represented by formula (I) to the at least one member selected from the group consisting of polyacrylic acids and salts thereof and the alkyl-modified carboxy polymer. Even when the skin emulsion composition contains an electrolyte, which is likely to decrease the viscosity, the present invention can also increase the thickening effect produced by at least one member selected from the group consisting of polyacrylic acids and salts thereof and an alkyl-modified carboxy polymer in the skin emulsion composition by adding a compound represented by formula (I).

This indicates that the present invention can provide a skin emulsion composition with higher viscosity, without adding an excessively increased amount of at least one member selected from the group consisting of polyacrylic acids and salts thereof or alkyl-modified carboxy polymer. Because it is unnecessary to add an excessively increased amount of at least one member selected from the group consisting of polyacrylic acids and salts thereof or alkyl-modified carboxy polymer, the present invention can provide a skin emulsion composition with higher viscosity without reducing the feeling of use, which is likely to be spoiled by, for example, stickiness.

The present invention can also significantly suppress the syneresis of a skin emulsion composition. Thus, the present invention can provide a markedly excellent skin emulsion composition exhibiting increased viscosity, with suppressed quality degradation that is caused by syneresis.

### (II) Viscosity Increasing Method

The present invention provides a method for increasing the viscosity of a skin emulsion composition comprising the following components (1), (2) and (4), the method comprising adding the following component (3) to the skin emulsion composition,
(1) at least one member selected from the group consisting of polyacrylic acids and salts thereof,
(2) an alkyl-modified carboxy polymer,
(3) a compound represented by the following formula (I) 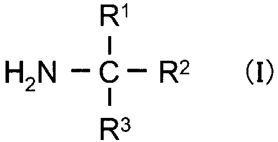 wherein R¹, R², and R³ are the same or different and represent C₁₋₃ alkyl or C₁₋₃ alkyl substituted with at least one hydroxyl group, with the proviso that at least one of R¹, R², and R³ is C₁₋₃ alkyl substituted with at least one hydroxyl group, and
(4) an oil in an amount of 10% by weight or more of the composition.

In this invention, components (1) to (4), the skin emulsion composition, the amounts of components (1) to (4), the production method of the skin emulsion composition, the target for application, the use and the method of application are as described above. Any other components optionally usable in the skin emulsion composition are also as described above.

The method according to the present invention can increase the thickening effect produced by at least one member selected from the group consisting of polyacrylic acids and salts thereof and an alkyl-modified carboxy polymer in a skin emulsion composition by applying a compound represented by formula (I) to the at least one member selected from the group consisting of polyacrylic acids and salts thereof and the alkyl-modified carboxy polymer. Even when the skin emulsion composition contains an electrolyte, which is likely to decrease the viscosity, the present invention can also increase the thickening effect produced by at least one member selected from the group consisting of polyacrylic acids and salts thereof and an alkyl-modified carboxy polymer by applying the compound represented by formula (I).

This indicates that the present invention can increase the viscosity of a skin emulsion composition, without adding an excessively increased amount of at least one member selected from the group consisting of polyacrylic acids and salts thereof or alkyl-modified carboxy polymer. Because it is unnecessary to add an excessively increased amount of at least one member selected from the group consisting of polyacrylic acids and salts thereof or alkyl-modified carboxy polymer, the present invention can increase the viscosity of the skin emulsion composition without reducing the feeling of use, which is likely to be spoiled by, for example, stickiness.

### Examples

The following Examples describe the present invention. Examples not covered by the scope of the claims are for illustrative purpose.

### Test Example 1

The components were mixed in accordance with the formulations shown in Table 1 below, thereby preparing a skin emulsion composition of Example 1. Specifically, while components 1, 2, and 5 to 8 shown in Table 1 were mixed, component 3 was added thereto to adjust the pH to 6.8 to 7.0, and the mixture was homogeneously mixed with a homogenizer (T.K. Homomixer, T.K. Robomix, Tokushu Kika Kogyo), thereby obtaining a gel. Components 9 and 10 were added to the obtained gel, and the pH was confirmed to be 6.8 to 7.0, followed by emulsification with a homogenizer. The viscosity of the obtained emulsion composition (Example 1) was measured.

Likewise, while components 1, 2, and 5 to 8 shown in Table 1 were mixed, component 4 was added thereto to adjust the pH to 6.8 to 7.0, and the mixture was homogeneously mixed with a homogenizer, thereby obtaining a gel. Components 9 and 10 were added to the obtained gel, and the pH was confirmed to be 6.8 to 7.0, followed by emulsification with a homogenizer. The viscosity of the obtained emulsion composition (Comparative Example 1) was measured.

The pH was measured with a pH meter F-52 (Horiba, Ltd.) at room temperature.

The viscosity was measured with a B-type viscometer. Specifically, the viscosity was measured at room temperature of 25°C with a BM-type viscometer (Tokyo Keiki Inc.) using a rotor No. 4 at 12 rpm for 60 seconds. The unit is mPa·s.

**Table 1**

| (Unit for Formulations: % by weight) | | | |
|---|---|---|---|
| Component | | Example 1 | Comparative Example 1 |
| 1 | Sodium Polyacrylate ^{*1} | 0.2 | 0.2 |
| 2 | Acrylates/C₁₀₋₃₀ Alkyl Acrylate Crosspolymer ^{*2} | 0.4 | 0.4 |
| 3 | Aminomethyl Propanediol | 0.446 | 0 |
| 4 | Potassium Hydroxide | 0 | 0.204 |
| 5 | Glycerol | 7 | 7 |
| 6 | Phenoxyethanol (Preservative) | 0.2 | 0.2 |
| 7 | Water | 74.89 | 74.72 |
| 8 | Etidronic Acid (Chelating Agent) | 0.08 | 0.08 |
| 9 | Cyclopentasiloxane ^{*3} | 10 | 10 |
| 10 | Liquid Paraffin ^{*4} | 5 | 5 |
| Total | | 100 | 100 |
| pH | | 6.8-7.0 | 6.8-7.0 |
| Viscosity (mPa·s at 25°C) | | 21,000 | 18,750 |

| | | | |
|---|---|---|---|
| *1 Rheogic 260H, Toagosei Co., LTD. *2 Pemulen TR-1, The Lubrizol Corporation *3 TSF405, Momentive Performance Materials Inc. *4 Moresco Violess U-8, Matsumura Sekiyu Kenkyujo | | | |

As is clear from Table 1, despite the same amounts of sodium polyacrylate and acrylates/C₁₀₋₃₀ alkyl acrylate crosspolymer contained in the emulsion compositions in Example 1 and Comparative Example 1, the emulsion composition prepared by using aminomethyl propanediol (Example 1 in Table 1) had a viscosity of 21,000 mPa·s, which is significantly improved as compared with the viscosity of 18,750 mPa·s of the emulsion composition prepared by using potassium hydroxide (Comparative Example 1 in Table 1). This indicates that the use of aminomethyl propanediol improves the thickening properties obtained by combining sodium polyacrylate with an acrylates/C₁₀₋₃₀ alkyl acrylate crosspolymer.

### Test Example 2

The components were mixed in accordance with the formulations shown in Table 2 below, thereby preparing a skin emulsion composition of Example 2. Specifically, component 5 was added to part of component 7 shown in Table 2, and then part of component 3 was added thereto until the pH reached 6.8 to 7.0 (mixture 1). Components 1, 2, 6, 8, and 9, the remainder of component 3, and the remainder of component 7 were mixed. After the pH was confirmed to be 6.8 to 7.0, the mixture was homogeneously mixed with a homogenizer (mixture 2). The obtained mixture 1 and mixture 2 were mixed, and heated to 60°C. Components 10 and 11, which had been heated to 60°C, were added thereto while the mixture was stirred, and emulsified with a homogenizer. The viscosity of the obtained emulsion composition (Example 2) was measured.

The procedure of Example 2 was repeated except that component 4 was used in place of component 3, thereby preparing an emulsion composition (Comparative Example 2). The viscosity of the obtained emulsion composition was measured.

The pH and viscosity were measured in the same manner as in Test Example 1.

**Table 2**

| (Unit for Formulations: % by weight) | | | |
|---|---|---|---|
| Component | | Example 2 | Comparative Example 2 |
| 1 | Sodium Polyacrylate ^{*1} | 0.4 | 0.4 |
| 2 | Acrylates/C₁₀₋₃₀ Alkyl Acrylate Crosspolymer ^{*2} | 0.4 | 0.4 |
| 3 | Aminomethyl Propanediol | 0.71 | 0 |
| 4 | Potassium Hydroxide | 0 | 0.29 |
| 5 | Electrolyte ^{*3} | 0.5 | 0.5 |
| 6 | Glycerol | 7 | 7 |
| 7 | Water | Balance | Balance |
| 8 | Chelating Agent | 0.08 | 0.08 |
| 9 | Preservative | 0.2 | 0.2 |
| 10 | Cyclopentasiloxane ^{*4} | 10 | 10 |
| 11 | Liquid Paraffin ^{*5} | 5 | 5 |
| Total | | 100 | 100 |
| pH | | 6.8-7.0 | 6.8-7.0 |
| Viscosity (mPa·s at 25°C) | | 20,000 | 15,750 |

| | | | |
|---|---|---|---|
| *1 Rheogic 260H, Toagosei Co., LTD. *2 Pemulen TR-1, The Lubrizol Corporation *3 Adenosine Monophosphate *4 TSF405, Momentive Performance Materials Inc. *5 Moresco Violess U-8, Matsumura Sekiyu Kenkyujo | | | |

As is clear from Table 2, even when an electrolyte, which is likely to reduce viscosity, was contained, the emulsion composition prepared by using aminomethyl propanediol (Example 2 in Table 2) had a viscosity of 20,000 mPa·s, which is significantly improved as compared with the viscosity of 15,750 mPa·s of the emulsion composition prepared by using potassium hydroxide (Comparative Example 2 in Table 2). This also indicates that the use of aminomethyl propanediol improves the thickening properties obtained by combining sodium polyacrylate with an acrylates/C₁₀₋₃₀ alkyl acrylate crosspolymer.

### Test Example 3

In the same manner as in Test Examples 1 and 2, skin emulsion compositions (Examples 3 to 8 and Comparative Examples 3 to 8) of formulations shown in Table 3 below were prepared. As a neutralizer, aminomethyl propanediol (AMPD) was used in Examples 3 to 8, and potassium hydroxide (KOH) was used in Comparative Examples 3 to 8. The viscosity of the obtained compositions was measured in the same manner as in the previous Test Examples.

**Table 3**

| (Unit for Formulations: % by weight) | | | | | | |
|---|---|---|---|---|---|---|
| | Ex. 3 | Ex. 4 | Ex. 5 | Ex. 6 | Ex. 7 | Ex. 8 |
| Sodium Polyacrylate | 0.4 | 0.4 | 0.4 | 0.4 | 0.4 | 0.4 |
| Acrylates/C₁₀₋₃₀ Alkyl Acrylate Crosspolymer | 0.4 | 0.4 | 0.4 | 0.4 | 0.4 | 0.4 |
| Glycerol | 7 | 7 | 7 | 7 | 7 | 7 |
| Liquid Paraffin | 0 | 3 | 6 | 9 | 12 | 15 |
| Phenoxyethanol (Preservative) | 0.2 | 0.2 | 0.2 | 0.2 | 0.2 | 0.2 |
| Etidronic Acid (Chelating Agent) | 0.05 | 0.05 | 0.05 | 0.05 | 0.05 | 0.05 |
| Potassium Hydroxide | 0 | 0 | 0 | 0 | 0 | 0 |
| Aminomethyl Propanediol | 0.41 | 0.41 | 0.41 | 0.41 | 0.41 | 0.41 |
| Water | Balance | Balance | Balance | Balance | Balance | Balance |
| Total | 100 | 100 | 100 | 100 | 100 | 100 |
| | | | | | | |

| | Comp. Ex. 3 | Comp. Ex. 4 | Comp. Ex. 5 | Comp. Ex. 6 | Comp. Ex. 7 | Comp. Ex. 8 |
|---|---|---|---|---|---|---|
| Sodium Polyacrylate | 0.4 | 0.4 | 0.4 | 0.4 | 0.4 | 0.4 |
| Acrylates/C₁₀₋₃₀ Alkyl Acrylate Crosspolymer | 0.4 | 0.4 | 0.4 | 0.4 | 0.4 | 0.4 |
| Glycerol | 7 | 7 | 7 | 7 | 7 | 7 |
| Liquid Paraffin | 0 | 3 | 6 | 9 | 12 | 15 |
| Phenoxyethanol (Preservative) | 0.2 | 0.2 | 0.2 | 0.2 | 0.2 | 0.2 |
| Etidronic Acid (Chelating Agent) | 0.05 | 0.05 | 0.05 | 0.05 | 0.05 | 0.05 |
| Potassium Hydroxide | 0.19 | 0.19 | 0.19 | 0.19 | 0.19 | 0.19 |
| Aminomethyl Propanediol | 0 | 0 | 0 | 0 | 0 | 0 |
| Water | Balance | Balance | Balance | Balance | Balance | Balance |
| Total | 100 | 100 | 100 | 100 | 100 | 100 |

As is clear from Fig. 1, the use of aminomethyl propanediol as a neutralizer exhibited clear improvement in viscosity of the emulsion compositions, as compared with the use of potassium hydroxide.

### Test Example 4

To examine the stability of an emulsion composition neutralized with aminomethyl propanediol, the skin emulsion composition prepared by using aminomethyl propanediol as a neutralizer and the skin emulsion composition prepared in the same manner by using potassium hydroxide as a neutralizer were analyzed as follows. 0.5 g of a sample (skin emulsion composition) was placed in a 1.5-mL tube centrifugal device (Nanosep MF 0.2 µm, Pall Corporation) equipped with a built-in centrifugal filter, and centrifuged with a centrifuge (Centrifuge 5415R, Eppendorf AG.) at 20°C, 16,100×g rcf for 5 minutes. Subsequently, the sample remained in the filter unit and the filtrate in the filtrate receiver were measured for weight, and evaluated for the likeliness of the occurrence of syneresis. A less decrease in the sample weight and a less increase in the filtrate weight indicated a more suppressed syneresis. The results reveal that this measurement method enables one to easily evaluate the syneresis of a composition, which was previously difficult to do.

The results also reveal that the emulsion composition prepared by using aminomethyl propanediol exhibited more suppressed syneresis of water than the emulsion composition prepared by using potassium hydroxide. The use of aminomethyl propanediol not only improves viscosity but also effectively suppresses syneresis; more specifically, the use of aminomethyl propanediol provided a stable emulsion composition having a desired viscosity and feeling of use, while exhibiting significantly suppressed quality degradation. To suppress syneresis, the results also suggest that it is preferable to increase the amount of oil, or add a plant-derived thickening agent, such as xanthan gum.

### Test Example 5

To investigate the efficacy of the composition, the following test was conducted. The skin emulsion composition used in the test contained 0.2% by weight of sodium polyacrylate, 0.6% by weight of an acrylates/C₁₀₋₃₀ alkyl acrylate crosspolymer, about 15% by weight of oil, about 1% by weight of aminomethyl propanediol, 5% by weight of ethanol, 0.5% by weight of adenosine monophosphate, 0.5% by weight of glyceryl glucoside (COSARTE-2G, Toyo Sugar Refining Co., Ltd.), 0.1% by weight of xanthan gum, a solvent, a chelating agent, an antioxidant, and a fragrance.

The test was conducted on human male subjects aged 35 to 50 years. The subjects applied about 0.4 g of the skin emulsion composition on half of their face twice a day in the morning and evening over a time period of 8 weeks, and the horny cell layer moisture content around the corners of the eyes and the depth of wrinkles around the corners of the eyes were evaluated. The evaluation was performed by the replica method in accordance with the guideline for evaluating anti-wrinkle products to obtain a novel efficacy issued by the Japanese Cosmetic Science Society. The results reveal that 8 weeks after the application of the composition, the part where the composition was applied exhibited a significant increase in moisture content in the horny cell layer and suppressed wrinkle formation, as compared with the part where the composition was not applied.

### Formulation Examples

The following shows formulation examples. In the formulation examples, the amount of components is denoted by the unit of % by weight.

### Formulation Example 1

Sodium Polyacrylate: 0.4
Acrylates/C₁₀₋₃₀ Alkyl Acrylate Crosspolymer: 0.4
Glycerol: 5
Dipropylene Glycol: 3
Phenoxyethanol: 0.2
Etidronic Acid: Suitable Amount
Aminomethyl Propanediol: Suitable Amount
Liquid Paraffin: 5
Cyclopentasiloxane: 10
Tocopherol: 0.1
Water: Balance
Total: 100
pH: 6.5 to 7

### Formulation Example 2

Sodium Polyacrylate: 0.4
Acrylates/C₁₀₋₃₀ Alkyl Acrylate Crosspolymer: 0.4
Glycerol: 4
Diglycerol: 2
Phenoxyethanol: 0.2
EDTA-2Na: Suitable Amount
Aminomethyl Propanol: Suitable Amount
Cyclopentasiloxane: 7
Dimethiconol: 0.2
Dimethicone: 2
Hydrogenated Polyisobutene: 5
Tocopherol: 0.1
Water: Balance
Total: 100
pH: 6.5 to 7

### Formulation Example 3

Sodium Polyacrylate: 0.2
Acrylates/C₁₀₋₃₀ Alkyl Acrylate Crosspolymer: 0.6
Glycerol: 5
Pentylene Glycol: 3
Phenoxyethanol: 0.2
Adenosine Phosphate: 0.3
Etidronic Acid: Suitable Amount
Aminomethyl Propanediol: Suitable Amount
Cyclopentasiloxane: 7
Hydrogenated Polyisobutene: 5
Macadamia Nut Oil: 2
Tocopherol: 0.1
Water: Balance
Total: 100
pH: 6.5 to 7

### Formulation Example 4

Sodium Polyacrylate: 0.2
Acrylates/C₁₀₋₃₀ Alkyl Acrylate Crosspolymer: 0.6
Glycerol: 5
Pentylene Glycol: 3
Phenoxyethanol: 0.2
Adenosine Phosphate: 0.5
Etidronic Acid: Suitable Amount
Aminomethyl Propanediol: Suitable Amount
PEG-20 Sorbitan Cocoate: 0.5
Hydrogenated Polyisobutene: 5
Isostearyl Neopentanoate: 2
Ethylhexyl Methoxycinnamate: 5
Octocrylene: 3
Tocopherol: 0.1
Water: Balance
Total: 100
pH: 6.5 to 7

### Formulation Example 5

Sodium Polyacrylate: 0.2
Acrylates/C₁₀₋₃₀ Alkyl Acrylate Crosspolymer: 0.6
Glycerol: 5
Pentylene Glycol: 3
Phenoxyethanol: 0.2
Adenosine Phosphate: 0.5
Etidronic Acid: Suitable Amount
Aminomethyl Propanediol: Suitable Amount
Cyclopentasiloxane: 5
Aminopropyl Dimethicone: 0.2
Hydrogenated Polyisobutene: 5
Isostearyl Neopentanoate: 2
Macadamia Nut Oil: 1
Tocopherol: 0.1
Water: Balance
Total: 100
pH: 6.5 to 7

## Claims

1. A skin emulsion composition comprising the following components (1) to (4), and having a viscosity of 10,000 mPa·s or more, with a BM-type viscometer using a rotor No.4 at 12 rpm for 60 seconds,
(1) at least one member selected from the group consisting of polyacrylic acids and salts thereof,
(2) an alkyl-modified carboxy polymer,
(3) a compound represented by the following formula (I) 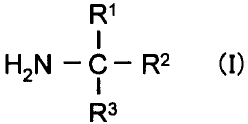 wherein R¹, R², and R³ are the same or different and represent C₁₋₃ alkyl or C₁₋₃ alkyl substituted with at least one hydroxyl group, with the proviso that at least one of R¹, R², and R³ is C₁₋₃ alkyl substituted with at least one hydroxyl group, and
(4) an oil in an amount of 10% by weight or more of the composition.

2. The skin emulsion composition according to claim 1, wherein the oil is at least one member selected from the group consisting of waxes, solid hydrocarbon oils, solid ester oils, liquid hydrocarbon oils, liquid ester oils, plant oils, animal oils, and silicone oils.

3. The skin emulsion composition according to any one of claim 1 or 2, comprising ethanol in an amount of 20% by weight or less of the composition.

4. The skin emulsion composition according to any one of Claims 1 to 3, wherein component (1) and component (2) are each present in an amount of 2% by weight or less of the composition.

5. The skin emulsion composition according to any one of Claims 1 to 4, which has a pH of 7 or less.

6. The skin emulsion composition according to any one of Claims 1 to 5, wherein component (2) is an acrylic acid/alkyl methacrylate copolymer.

7. The skin emulsion composition according to any one of Claims 1 to 6, wherein component (3) is at least one member selected from the group consisting of aminomethyl propanol, aminomethyl propanediol, and amino hydroxymethyl propanediol.

8. The skin emulsion composition according to any one of Claims 1 to 7, excluding compositions for application to scalp hair and/or scalp.

9. The skin emulsion composition according to any one of Claims 1 to 8, further comprising an electrolyte, the composition being for use in moisturizing skin, preventing wrinkles on skin, or whitening skin.

10. A method for increasing viscosity of a skin emulsion composition comprising the following components (1), (2) and (4), the method comprising adding the following component (3) to the skin emulsion composition,
(1) at least one member selected from the group consisting of polyacrylic acids and salts thereof,
(2) an alkyl-modified carboxy polymer,
(3) a compound represented by the following formula (I) 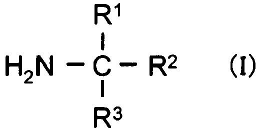 wherein R¹, R², and R³ are the same or different and represent C₁₋₃ alkyl or C₁₋₃ alkyl substituted with at least one hydroxyl group, with the proviso that at least one of R¹, R², and R³ is C₁₋₃ alkyl substituted with at least one hydroxyl group; and
(4) an oil in an amount of 10% by weight or more of the composition.

## Patentansprüche

1. Hautemulsionszusammensetzung, umfassend die folgenden Komponenten (1) bis (4) und mit einer Viskosität von 10.000 mPa·s oder mehr, gemessen mit einem Viskosimeter vom BM-Typ mit einem Rotor Nr. 4 bei 12 U/min über 60 Sekunden,
(1) mindestens ein aus der aus Polyacrylsäuren und Salzen davon bestehenden Gruppe ausgewählte Element,
(2) ein alkylmodifiziertes Carboxypolymer,
(3) eine durch die folgende Formel (I) wiedergegebene Verbindung 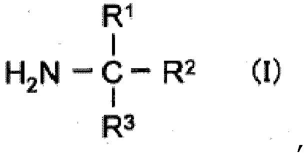 wobei R¹, R² und R³ gleich oder verschieden sind und für C₁₋₃-Alkyl oder durch mindestens eine Hydroxylgruppe substituiertes C₁₋₃-Alkyl stehen, mit der Maßgabe, dass mindestens einer der Reste R¹, R² und R³ für durch mindestens eine Hydroxylgruppe substituiertes C₁₋₃-Alkyl steht, und
(4) ein Öl in einer Menge von 10 Gew.-% oder mehr der Zusammensetzung.

2. Hautemulsionszusammensetzung nach Anspruch 1, wobei es sich bei dem Öl um mindestens ein aus der aus Wachsen, festen Kohlenwasserstoffölen, festen Esterölen, flüssigen Kohlenwasserstoffölen, flüssigen Esterölen, Pflanzenölen, tierischen Ölen, und Siliconölen bestehenden Gruppe ausgewähltes Element handelt.

3. Hautemulsionszusammensetzung nach einem der Ansprüche 1 oder 2, welche Ethanol in einer Menge von 20 Gew.-% oder weniger der Zusammensetzung umfasst.

4. Hautemulsionszusammensetzung nach einem der Ansprüche 1 bis 3, wobei die Komponente (1) und die Komponente (2) jeweils in einer Menge von 2 Gew.-% oder weniger der Zusammensetzung vorliegen.

5. Hautemulsionszusammensetzung nach einem der Ansprüche 1 bis 4 mit einem pH-Wert von 7 oder weniger.

6. Hautemulsionszusammensetzung nach einem der Ansprüche 1 bis 5, wobei es sich bei der Komponente (2) um ein Acrylsäure-Alkylmethacrylat-Copolymer handelt.

7. Hautemulsionszusammensetzung nach einem der Ansprüche 1 bis 6, wobei es sich bei der Komponente (3) um mindestens ein aus der aus Aminomethylpropanol, Aminomethylpropandiol und Aminohydroxymethylpropandiol bestehenden Gruppe ausgewähltes Element handelt.

8. Hautemulsionszusammensetzung nach einem der Ansprüche 1 bis 7, mit Ausnahme von Zusammensetzungen zur Anwendung auf dem Kopfhaar und/oder der Kopfhaut.

9. Hautemulsionszusammensetzung nach einem der Ansprüche 1 bis 8, welche weiterhin einen Elektrolyten umfasst, wobei die Zusammensetzung zur Verwendung beim Befeuchten der Haut, beim Verhindern von Falten auf der Haut oder beim Aufhellen der Haut bestimmt ist.

10. Verfahren zum Erhöhen der Viskosität einer Hautemulsionszusammensetzung, die die folgenden Komponenten (1), (2) und (4) umfasst, wobei das Verfahren die Zugabe der folgenden Komponente (3) zu der Hautemulsionszusammensetzung umfasst,
(1) mindestens ein aus der aus Polyacrylsäuren und Salzen davon bestehenden Gruppe ausgewählte Element,
(2) ein alkylmodifiziertes Carboxypolymer,
(3) eine durch die folgende Formel (I) wiedergegebene Verbindung 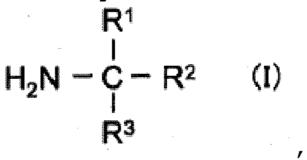 wobei R¹, R² und R³ gleich oder verschieden sind und für C₁₋₃-Alkyl oder durch mindestens eine Hydroxylgruppe substituiertes C₁₋₃-Alkyl stehen, mit der Maßgabe, dass mindestens einer der Reste R¹, R² und R³ für durch mindestens eine Hydroxylgruppe substituiertes C₁₋₃-Alkyl steht, und
(4) ein Öl in einer Menge von 10 Gew.-% oder mehr der Zusammensetzung.

## Revendications

1. Composition d'émulsion pour la peau, comprenant les composants (1) à (4) suivants, et ayant une viscosité de 10 000 mPa.s ou plus, avec un viscosimètre de type BM en utilisant un rotor n°4 à 12 tours/min pendant 60 secondes,
(1)au moins un élément choisi dans le groupe constitué par les acides polyacryliques et les sels de ceux-ci,
(2)un polymère carboxy à modification alkyle,
(3)un composé représenté par la formule (I) suivante 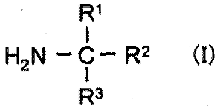 où R¹, R² et R³ sont identiques ou différents et représentent C₁₋₃-alkyle ou C₁₋₃-alkyle substitué par au moins un groupement hydroxyle, à condition qu'au moins l'un parmi R¹, R² et R³ soit C₁₋₃-alkyle substitué par au moins un groupement hydroxyle, et
(4)une huile selon une quantité de 10% en poids ou plus de la composition.

2. Composition d'émulsion pour la peau selon la revendication 1, dans laquelle l'huile est au moins un élément choisi dans le groupe constitué par les cires, les huiles hydrocarbonées solides, les huiles d'ester solides, les huiles hydrocarbonées liquides, les huiles d'ester liquides, les huiles végétales, les huiles animales, et les huiles de silicone.

3. Composition d'émulsion pour la peau selon l'une quelconque des revendications 1 ou 2, comprenant de l'éthanol selon une quantité de 20% en poids ou moins de la composition.

4. Composition d'émulsion pour la peau selon l'une quelconque des revendications 1 à 3, dans laquelle le composant (1) et le composant (2) sont chacun présents selon une quantité de 2% en poids ou moins de la composition.

5. Composition d'émulsion pour la peau selon l'une quelconque des revendications 1 à 4, qui possède un pH de 7 ou moins.

6. Composition d'émulsion pour la peau selon l'une quelconque des revendications 1 à 5, dans laquelle le composant (2) est un copolymère d'acide acrylique/ méthacrylate d'alkyle.

7. Composition d'émulsion pour la peau selon l'une quelconque des revendications 1 à 6, dans laquelle le composant (3) est au moins un élément choisi dans le groupe constitué par l'aminométhyl propanol, l'amino-méthyl propanediol, et l'aminohydroxyméthyl propanediol.

8. Composition d'émulsion pour la peau selon l'une quelconque des revendications 1 à 7, à l'exclusion des compositions destinées à une application aux cheveux et/ou au cuir chevelu.

9. Composition d'émulsion pour la peau selon l'une quelconque des revendications 1 à 8, comprenant en outre un électrolyte, la composition étant destinée à une utilisation pour hydrater la peau, empêcher les rides sur la peau, ou blanchir la peau.

10. Méthode destinée à augmenter la viscosité d'une composition d'émulsion pour la peau, comprenant les composants (1), (2) et (4) suivants, la méthode comprenant l'addition du composant (3) suivant à la composition d'émulsion pour la peau,
(1)au moins un élément choisi dans le groupe constitué par les acides polyacryliques et les sels de ceux-ci,
(2)un polymère carboxy à modification alkyle,
(3)un composé représenté par la formule (I) suivante 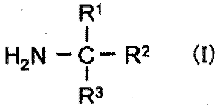 où R¹, R² et R³ sont identiques ou différents et représentent C₁₋₃-alkyle ou C₁₋₃-alkyle substitué par au moins un groupement hydroxyle, à condition qu'au moins l'un parmi R¹, R² et R³ soit C₁₋₃-alkyle substitué par au moins un groupement hydroxyle, et
(4)une huile selon une quantité de 10% en poids ou plus de la composition.
